# EUROPEAN PATENT SPECIFICATION

(11) **EP 1 292 828 B1**
(45) Date of publication and mention of the grant of the patent: **13.10.2004**
(21) Application number: 01940836.8
(22) Date of filing: 22.06.2001
(51) Int. Cl.: G01N 33/531, G01N 33/94, G01N 30/48

(54) **PARTICLES AND THEIR USE IN MOLECULAR IMPRINTING**
PARTIKEL UND IHRE VERWENDUNG IN MOLEKULARER Prägung
PARTICULES ET LEUR UTILISATION DANS L'EMPREINTE MOLECULAIRE

(30) Priority: 23.06.2000 GB 0015449
(43) Date of publication of application: 19.03.2003
(73) Proprietor: Prometic Biosciences Ltd, Ballasalla, Isle of Man IM9 2AP (GB)
(72) Inventor: CARTER, Steven Robert, Brook Hill Sheffield S3 7HF (GB); RIMMER, Steven, Brook Hill Sheffield S3 7HF (GB)
(74) Representative: Perry, Robert Edward
(86) International application number: PCT/GB2001/002794
(87) International publication number: WO 2001/098784

(56) References cited:
- WO-A-01/19886
- GLAD M ET AL: "MOLECULARLY IMPRINTED COMPOSITE POLYMERS BASED ON TRIMETHYLOLPROPANE TRIMETHACRYLATE (TRIM) PARTICLES FOR EFFICIENT ENANTIOMERIC SEPARATIONS" REACTIVE POLYMERS, ELSEVIER SCIENCE PUBLISHERS, AMSTERDAM, NL, vol. 25, no. 1, 1 May 1995 (1995-05-01), pages 47-54, XP000505034 ISSN: 0923-1137
- GLAD M ET AL: "USE OF SILANE MONOMERS FOR MOLECULAR IMPRINTING AND ENZYME ENTRAPMENT IN POLYSILOXANE-COATED POROUS SILICA" JOURNAL OF CHROMATOGRAPHY, vol. 347, no. 1, 1985, pages 11-24, XP002183900 ISSN: 0021-9673
- VIDYASANKAR, SUNDARESAN ET AL: "Molecularly imprinted ligand-exchange adsorbents for the chiral separation of underivatized amino acids" J. CHROMATOGR., A (1997), 775(1 + 2), 51-63 , XP002183901
- VIDYASANKAR SUNDARESAN ET AL: "Selective ligand-exchange adsorbents prepared by template polymerization." BIOTECHNOLOGY AND BIOENGINEERING, vol. 48, no. 5, 1995, pages 431-436, XP002183902 ISSN: 0006-3592

## Description

### Field of the Invention

This invention relates to particles and their use in molecular imprinting. More particularly, this invention relates to particles suitable for use as separation media, and to their preparation by molecular imprinting.

### Background to the Invention

Many methods are known, to achieve separation and analysis of a target molecule, from an impure mixture, for example, liquid chromatography-mass spectroscopy (LC-MS) and high performance liquid chromatography (HPLC). Both these methods are non-specific. That is, certain parameters (such as UV wavelength and flow rate) must be set and maintained, in order to identify a particular compound.

Molecular imprinting can be used to produce polymers that are selective for specific target molecules. The product is manufactured in a series of stages. Firstly, selected copolymerisable monomers are mixed with the target molecule. Secondly, the mixture undergoes polymerization, whereby the target molecule is trapped within the polymer. Thirdly, the target molecule is extracted, leaving behind a cavity, or imprint, corresponding to the target molecule. The resultant polymeric product is, therefore, selective for the target molecule.

There are, however, a number of disadvantages with this technique. In most cases, polymerization of the monomers and the target molecule results in a solid mass which must be broken and ground, e.g. in a mill, to provide particles of an appropriate size. By this process, it is difficult to produce particles having a uniform "shape", e.g. as are typically required for use in separation tubes or columns, in order to avoid significant back-pressure in use. HPLC columns, for example, require particles having a narrow range of diameters, typically 80% of particles with ±20% of mean diameter. Therefore, if relatively uniform particles are required, the particles are sieved. The use of any or all of these processes results in large losses of material, as "fines". For example, the breaking and grinding may result in 50% loss of the prepared material, and sieving may result in loss of as much as 95% of the prepared material. Obviously, this is a major economic disadvantage.

Another disadvantage of current techniques is that a high proportion of the target molecule is usually trapped within the polymer matrix. It is generally understood that, whilst some target can be removed (e.g. by washing with organic solvent), a proportion will remain trapped in the matrix.

This has two undesirable effects. Firstly, target molecules are often expensive. They may, for example, be drug candidates, specifically manufactured to test their efficacy. In such cases, it is desirable to remove and reuse the target molecule. Secondly, any target molecules remaining in the matrix adversely affect its utility in trace analysis and in quantitative analysis. The imprint molecules will "leak" out, and mask the adsorption of the target molecule.

This is a serious problem, and one which has been addressed in the prior art. A strategy has evolved which utilizes an analogue of the target molecule, as the imprint. If the analogue forms a cavity which can be selectively filled by the target molecule, and the analogue can be separated from the target molecule by, for example, HPLC, then matrices may be usable in trace and quantitative assays.

Yet another disadvantage arises from the, typically, low water-solubility of the monomers. Consequently, polymerization is carried out in organic solvent. Aside from the higher cost of organic solvents as compared to water, and the increased environmental concerns about the use of organic solvents, an organic system does not correspond to biological systems. Many bioassays for which imprinted polymers could be useful should be conducted in water, in order to represent a natural system. It is widely acknowledged that replacement of organic solvent with water is not feasible, due to the high degree of cross-linking required to maintain the cavity shape.

Glad et al, Reactive Polymers, vol. 25, no. 1, 1995, pages 47-54, describes the manufacture of spherical microparticles, comprising a shell of molecularly imprinted cross-linked methacrylate polymers. A mixture of methacrylic acid, ethylene glycol dimethacrylate and an imprinting analyte are added to TRIM particles and polymerised. The imprinted composite particles are washed. Chromatography using the template molecule as the analyte is performed.

Glad et al, J. Chromatography, vol. 347, no. 1, 1985, pages 11-24, describes the use of organic silane monomers in the preparation of substrate-selective polymers by molecular imprinting. Silanes are allowed to polymerise on the surface of porous silica particles in aqueous solution in the presence of a dye substrate. The resulting particles are washed thoroughly. The polysiloxane-coated particles are evaluated in HPLC experiments, using an aqueous solvent.

Sundaresan et al, J. Chromatography, A (1997), 775(1+2), 51-63, and also in Biotechnology and Bioenginneering, vol. 48, no. 5, 1995, pages 431-436, described imprinted polymers grafted to derivatised silica particles or TRIM particles, and their use for the detection of analytes.

### Summary of the Invention

According to the present invention, a particle capable of specific binding to an imprint molecule, has an outer shell including cavities that correspond to the imprint molecule, and an inner core substantially free of such cavities. The particle also includes an amphipathic material.

Such particles may be manufactured by the steps of:
a) polymerising a mixture of the imprint molecule and polymerisable material, around the core, to form a shell containing the imprint molecule; and
b) removing the imprint molecule, e.g. by washing, to leave a corresponding cavity.

The invention advantageously allows selection assays to be performed in an aqueous environment A further advantage is that particles of the invention may readily be prepared, having a uniform size. The invention provides a generic method for separating molecules on the basis of their shape, size and chemical composition.

### Description of the Invention

Particles of the present invention are manufactured from materials which may be known *per se*. There are, essentially, 2 stages for manufacturing the particles. Firstly, a mixture of polymerisable material and imprint molecule (hereinafter called the polymerisable mixture) is polymerised, around a core, to form a shell containing the imprint molecule. Secondly, the imprint molecule is removed, leaving behind imprint specific cavities.

The core of the particle may be commercially available, or it may be manufactured *in situ*. For example, it may be formed from polymerisable monomers such as divinylbenzene (DVB)/styrene.

The shell is formed from any suitable polymerisable material. This may be, for example, methyl methacrylate (MMA) or EGDMA, or a combination of these. An imprint molecule is then added, which may be any suitable ligand, which it is desired to test for and, finally, the imprint molecule is washed off. The washing solution may be, for example, a solution of water/acetone, H₃PO₄, or methanol.

In order to enhance its wetting properties, the particle has polar groups on its outer surface, by including an amphipathic molecule in the particle. Such amphipathic molecules also serve to enhance pore formation and non-covalent bonding interactions with the imprinted molecule. The amphipathic molecule may be included in the polymerisable mixture, or the core may be formed around a "molecular scaffold" which comprises the amphipathic molecule. For example, oleyl phenyl hydrogen phosphate (OPHP), an amphipathic molecule, may be included in the polymerisable mixture, or may form a micelle around which the core is formed.

Particles of the invention are typically between 10, 50 and 100 times smaller or larger in size than the Example herein, and preferably have a size distribution similarly related to the Example. The shell typically constitutes up to 10, 20, 30, 40 or 50% of the cross-sectional dimension of the particle, and may be between 10, 50 and 100 times thinner or thicker than the Example herein. The thinner the shell, the more easily the imprint molecules can be washed out, due to their proximity to the outer surface.

The invention will now be described by way of example only with reference to the accompanying drawings, in which:
Figure 1 is a schematic diagram showing a sequence of steps to produce particles;
Figure 2 provides schematic diagrams of two alternative sequences of steps to produce particles;
Figure 3 shows the structures of two compounds useful in manufacturing the particles;

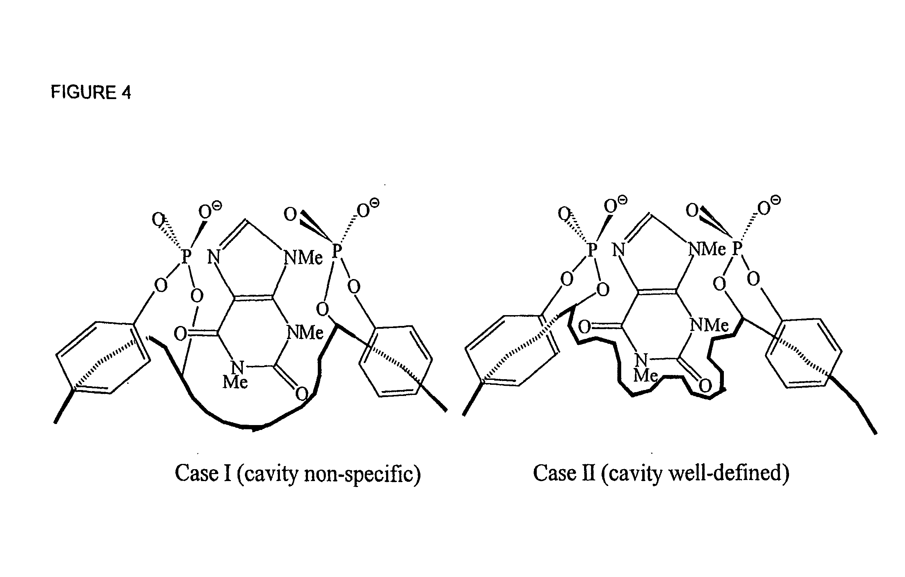

Figure 4 is a schematic illustration of non-specific and specific cavities that may be formed by the invention; and
Figure 5 illustrates the effect of pH on binding of caffeine and theophylline to imprinted core-shell particles.

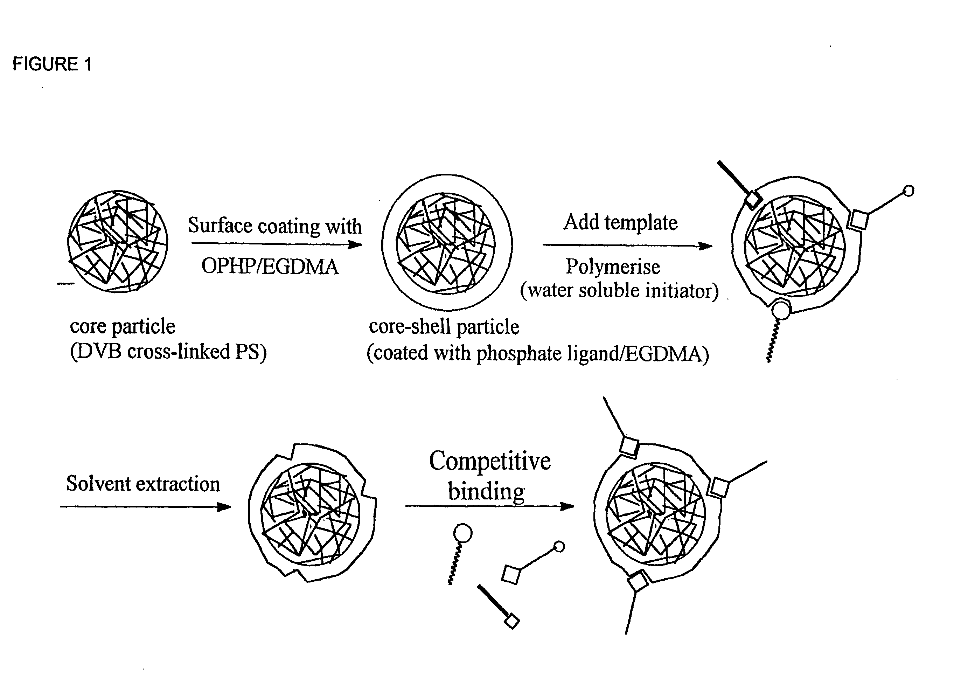

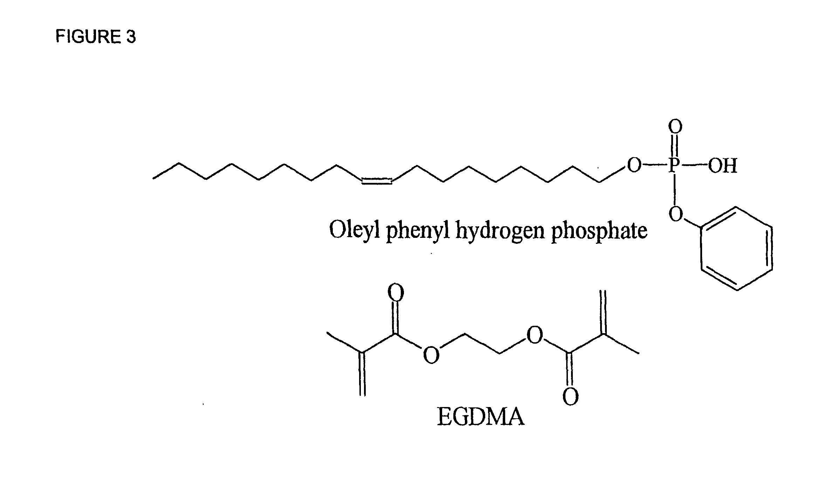

Referring to Fig. 1, a cross-linked polystyrene core is manufactured from styrene and DVB. This is coated with a combination of OPHP and EGDMA (Fig. 3), to produce a "core-shell" particle. Subsequently, a template molecule, or imprint molecule, is added, and this is followed by polymerisation of the EGDMA with a water soluble initiator. The imprint molecules are removed by solvent extraction, e.g. using acetone, leaving target specific cavities in the shell of the particle.

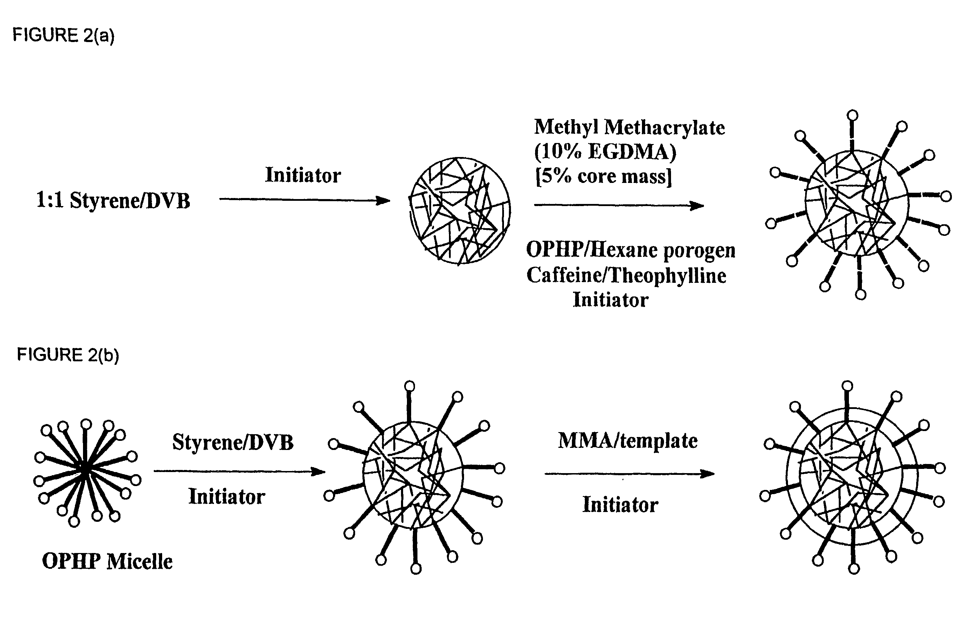

Referring to Fig. 2, the core may be manufactured from a mixture of styrene and DVB, and the shell may be manufactured from MMA/EGDMA. OPHP is included, to provide polar surface groups on the particle. Alternatively, a styrene/DVB core may be formed around an OPHP micelle, and then MMA may be added to form the outer shell.

The following Examples illustrate the invention.

### Example 1

### i. Preparation of Divinylbenzene cross-linked Polystyrene core particles

An aqueous buffer solution of 4-morpholineethanesulphonic acid-monohydrate (0.6398 g, 3.00 mmol, 50 mM) in deionised water (52.5 ml) was prepared then purged with nitrogen for 15 min. Sodium dodecyl sulphate [source BDH, 'specially pure'] (1.50 g, 5.20 mmol) was added then the mixture stirred for dissolution, sonicated for 10 min at room temperature then adjusted to pH 6.0 by the addition of 1M NaOH. The solution was stirred at 300-350 r.p.m. under a nitrogen atmosphere with the temperature maintained at 60°C, and a styrene (4.0173 g, 38.57mmol)/dinvinylbenzene (5.0217 g, 38.57 mmol) mixture was then added dropwise to the mixture over 30 min. The polymerisation was initiated by the addition of one portion of 4,4'-azobis-(4-cyanovaleric acid) (0.5460 g, 1.95 mmol) and stirring continued for 16 hour to give an emulsion of core particles.

### ii. Surface Molecular Imprinting

A mixture of ethylene glycol dimethacrylate (0.4520 g, 2.28 mmol) 15% by massl ml) was added to the emulsion at 60°C and stirring continued for 30 min. A caffeine imprint was obtained by adding caffeine (0.3735 g; 1.92 mmol; 2.0 mole equivalents) and stirring continued for 30 min to reach equilibration, then surface polymerisation was initiated by the addition of 4,4'-azobis-(4-cyanovaleric acid) (0.5460 g, 1.95 mmol) in one portion. The reaction was continued for 105 min at 60°C then quenched by lowering the temperature to 0°C using an ice-water bath.

Stable emulsions were formed when the percentage mass of EGDMA in the coat thickness was both increased to 20% and reduced to 1%, using the standard methodology described above for the surface molecular imprinting of polymer colloid particles with caffeine.

### Example 2

A variety of surface molecularly-imprinted core-shell particles was prepared according to the procedure given in Example 1, except that caffeine was replaced by alternative imprint molecules, including theophylline, codine, morphine, piperazine, imidazole, harmine, carboline, propranolol and atenolol, at relative concentrations in the range 1.0 to 2.0 mole equivalents. In all cases, stable emulsions of imprinted core-shell particles were obtained.

### Example 3

### Synthesis of core-shell particles with a low cross-link density shell

Particles with a total shell mass of 5% w/w consisting of methyl methacrylate (90%) and EGDMA (10%) were prepared as shown in Figure 2(a).

### i. Preparation of Divinylbenzene-cross-linked Polystyrene core particles

An aqueous solution of 4-morpholineethanesulphonic acid monohydrate (4.265g, 20 mmol, 50 mM) in deionised water (350 ml) was prepared then purged with nitrogen for 15 min. Sodium dodecyl sulphate (10.0 g, 34.6 mmol) was added and the mixture stirred, sonicated for 10 min at room temperature then adjusted to pH 6.0 by the addition of 1M NaOH. The solution was stirred at 300-350 r.p.m. under a nitrogen atmosphere with the temperature maintained at 60°C, and a styrene (26.782 g, 0.257 mol)/divinylbenzene (33.478 g, 0.257 mol) mixture was then added dropwise to the mixture over 30 min. The polymerisation was initiated by the addition of one portion of 4,4'-azobis-(4-cyanovaleric acid) (3.640 g, 11.8 mmol) and stirring continued for 16 h to give an emulsion of core particles.

### ii. Surface molecular imprinting to form particles with low cross-link density shell

A mixture of methyl methacrylate (3.013 α. 30.1 mmol). oleyl phenyl hydrogen hexane (1.0 g) in water (50 ml) was then added and stirring continued for 30 min. The template molecule*, in this instance caffeine (3.883 g, 20 mmol, 2.0 mol equiv. with respect to OPHP), was added and stirring continued for 30 min to reach equilibration, then surface polymerisation was initiated by the addition of 4,4'-azobis-(4-cyanovaleric acid) (3.64 g, 11.8 mmol) in one portion. The reaction was continued for 105 min at 60°C then quenched by reducing the temperature to 0°C using an ice-water bath.

### Example 4

### Synthesis of core-shell particles with a Divinylbenzene/oleyl phenyl hydrogen phosphate-cross-linked core

### i. Preparation of Divinylbenzene cross-linked polystyrene /oleyl phenyl hydrogen core particles

An aqueous solution of 4-morpholineethanesulphonic acid-monohydrate (0.4265 g, 2 mmol) in deionised water (35 ml) was prepared then purged with nitrogen for 15 min. Sodium dodecyl sulphate (1.0 g, 3.46 mmol) was added and the mixture stirred, sonicated for 10 min at room temperature then adjusted to pH 6.0 by the addition of 1 M NaOH. The solution was stirred at 300-350 r.p.m. under a nitrogen atmosphere with the temperature maintained at 60°C. Oleyl phenyl hydrogen phosphate (0.4260 g, 1.0 mmol) was added and stirring continued for 30 min. A mixture of styrene (2.677 g, 25.7 mmol) and divinylbenzene (3.349 g, 25.7 mmol) mixture was added dropwise over 30 min. Polymerisation was initiated by the addition of one portion of 4,4'-azobis-(4-cyanovaleric acid) (0.3640 g, 1.18 mmol) and stirring continued for 6 h.

### ii. Surface molecular imprinting with methyl methacrylate

A suspension of methyl methacrylate (0.3013 g, 3.0 mmol) in deionised water (5.0 ml) was added to the emulsion at 60°C and stirring continued for 30 min. The template compound, in this instance caffeine (0.3883 g, 2.0 mmol) was added and stirring continued for 30 min to reach equilibration, whereupon surface polymerisation was initiated by the addition of 4,4'-azobis-(4-cyanovaleric acid) (0.3640 g, 1.18 mmol) in one portion. The reaction was continued for 105 min at 60°C then quenched by lowering the temperature to 0°C using an ice-water bath. These reactions are shown in Figure 2(b).

### Example 5

### Preparation of imprinted core-shell particles for binding studies

A 2.0 ml aliquot of core-shell particle emulsion was stirred rapidly at room temperature and acetone (1.4 -1.8 ml) added dropwise over a 2 min period until a thick suspension was achieved. The suspension was poured into a 5.0 ml capacity SEC housing (pre-inserted in a vacuum manifold) and allowed to settle for 30 min then drained under vacuum. A 2:1 acetone/deionised water solution (2.0 ml) was added to the SEC cartridge, stirred to form a suspension then drained. This procedure was further repeated (6 x 2.0 ml) until no more SDS could be detected in the filtrate by U.V. absorption spectroscopy and no residual template could be detected by LC-MS. The SEC's were fitted with 20 ml solvent reservoirs and washed sequentially according to the protocol given in Table 1. Following this procedure the imprinted resins were either used directly or air-dried and stored for future use.

As an alternative to washing by use of filtration, imprinted polymer particles of the invention were precipitated with isopropyl alcohol and isolated by centrifugation. Washing was performed by re-suspending the particles in the solvents described in Table 1 and isolating the particles after each wash by centrifugation. This procedure was found to be an especially quick and useful way of washing the imprinted polymer particles.

**Table 1:**

| washing protocol for preparation of imprinted resins | | | |
|---|---|---|---|
| Wash No. | Eluant | Volume/ml | Comments |
| 1 | 2:1 acetone/water | 7 x 2.0 | To determine excess template |
| 2 | 2:1 acetone/water | 2 x 20.0 | Washing off excess unbound |
| 3 | 1M N₃PO₄ | 2 x 20.0 | Washing off bound template |
| 4 | Water | 2 x 2.0 | Washing off acid |
| 5 | MeOH | 2 x 20.0 | Non-specific eluant wash |
| 6 | Water | 2 x 2.0 | Washing off methanol |
| 7 | 1M H₃PO₄ | 1 x 2.0 | For equilibration |
| 8 | Water | 1 x 2.0 | For aqueous binding studies |

### Example 6

### Binding of caffeine and theophylline to imprinted core-shell particles

Imprinted and non-imprinted core-shell particles (2.0 ml) synthesised as described in Examples 1 and 2 and prepared for use as described in Example 5 were added to a 220 ml capacity ultracentrifugation cartridge containing a 100,000 Dalton cut-off membrane and precipitated with 3 ml of isopropanol. Samples were washed sequentially with 70% IPA/water (3 x 15 ml), 1M H₃PO₄ (2 x 20 ml); methanol (2 x 20 ml) and water (2 x 20 ml) with centrifugation at 10°C, 8000 x g between washes.

An aqueous 1:1 mixture of caffeine (57.5 µg/ml) and theophylline (53.5 µg/ml) was prepared and adjusted to pH 7.0. Samples (2.0 ml) were added to washed caffeine-imprinted, theophylline-imprinted and non-imprinted core-shell particles and the suspensions thoroughly mixed and allowed to stand for 30 min with repeated agitation every 10 min. The mixtures were drained under vacuum, filtered and the supernatants analysed by LC-MS analysis using a Waters 2690 separations module and a Micromass LCZ Platform operating in ESI mode with a 4000 Dalton range quadruple mass analyser. The fractional uptake (U) was determined by peak area U = (A₁-A₂)/A₁ where A₁ = peak area of solution before application to resin and A₂=peak area of supernatant after contact with the resin. The results of these binding studies are reported in Table 2.

**Table 2:**

| Fractional uptake of caffeine/theophylline for polymer imprinted resins | | | | | | |
|---|---|---|---|---|---|---|
| Coating thickness/% mass of core mass and depth/nm | Fractional uptake (U) by imprinted resin | | | | | |
| | P (caff) | | P (theo) | | P (blank) | |
| | *Ucaff* | *Utheo* | *Ucaff* | *Utheo* | *Ucaff* | *Utheo* |
| 5% (10 nm) | 0.94 | 0.51 | 0.57 | 0.48 | 0.56 | 0.51 |
| 1% (2 nm) | 0.59 | 0.72 | 0.81 | 0.91 | 0.73 | 0.88 |
| 20% (40 nm) | 0.85 | 0.95 | 0.92 | 0.95 | 0.89 | 0.98 |

It was found from these studies that selectivity for caffeine uptake over theophylline was only observed when the shell thickness was an optimal value of 5% (i.e. the mass of EGDMA used was 5% of the core particle mass), which also represents a layer thickness of 10 nm-equivalent to the molecular dimensions of the template molecule used, in this instance caffeine or theophylline.

No selectivity for caffeine is observed when the shell is 20% of the core-mass, suggesting that complete encapsulation of the template molecules by cross-linked EGDMA alone is occurring at the particle surface, that is with no participation of the phosphate head groups.

No selectivity for caffeine is observed when the shell is 1% of the core-mass, and it was also observed that the uptake of both caffeine and theophylline was 14-20% lower for the caffeine-templated resin than the theophylline- and blank-templated resins. The lack of selectivity suggests that hydrophilic binding forces from the phosphate head groups dominate any hydrophobic interactions at the colloid surface, due to the shallow layer thickness.

### Example 7

### Effect of pH on Competitive Binding to Imprinted Resins

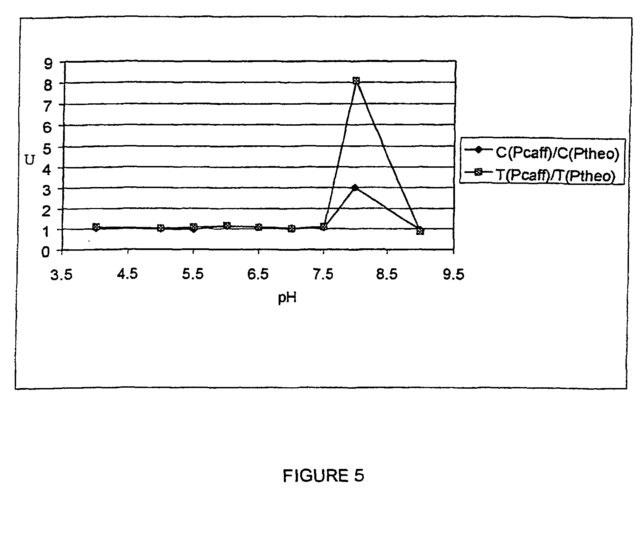

The effect of pH on competitive binding of a 1:1 caffeine/theophylline mixture (57.5/53.5 µg/ml) in 0.1 M sodium phosphate buffer to caffeine and theophylline imprinted core-shell particles and blank-templated resins was determined in the pH range 4.0-9.0. Three series of solid-phase extraction cartridges (9 each of caffeine- and theophylline- templated resins and blank-resins), each containing 300 mg of resin, were prepared according to Example 5. Solutions of 1:1 caffeine/theophylline (57.5/53.5 µg/ml) in 0.1 M sodium phosphate bufferwere prepared in the pH range 4.0-9.0. These solutions were analysed for caffeine and theophylline content, both before and after application to the resin, according to the method described in Example 6. The uptake of caffeine (U_{c}) from the 1:1 caffeine/theophylline solutions in the pH range 4.0-9.0 was determined for the caffeine-imprinted resin [P(caff)], the theophylline-imprinted resin [P(theo)] and for the blank resins. Similarly, the uptake of theophylline (Uₜ) from the 1:1caffeine/theophylline solutions in the pH range 4.0-9.0 was determined for the caffeine-imprinted resin [P(caff)], the theophylline-imprinted resin [P(theo)] and for the blank resins. Uptake values (relative to blank) were plotted against pH as shown in Figure 5.

These studies demonstrate that (i) selective uptake of caffeine from a 1:1 caffeine/theophylline mixture is more pronounced for the caffeine-templated resin than the theophylline-templated resin in sodium phosphate buffer at pH 8.0.; (ii) that selective uptake of theophylline from a 1:1 caffeine/theophylline mixture is more pronounced for the caffeine-templated resin than the theophylline-templated resin in sodium phosphate buffer at pH 8.0; and (iii) that selective uptake of theophylline on a caffeine-templated resin is more pronounced than the selective uptake of caffeine on a caffeine-templated resin in sodium phosphate buffer at pH 8.0.

### Example 8

### Investigation into the selectivity of molecularly-imprinted core-shell particles using 1:1 mixtures of structurally analogous components

Various core-shell particles were surface-molecularly imprinted with water soluble compounds according to the method described in Example 1. Aqueous 1:1 mixtures of the imprinting molecule and a structurally analogous compound were prepared and the solutions adjusted to pH 7.0 with 1 M NaOH/1M HCl. The content of each component in the mixture, both before and after application to the resins, was carried out according to the method described in Example 6. The fractional uptake values (U₁ and U₂) for each component in the 1:1 mixture after application to the resin, were determined for resins (P) imprinted with each of the analogous compounds 1 and 2, and also for the blank (non-imprint) resin. The results are summarised in Table 3 and demonstrate enhanced selectivity for the molecule used for imprint formation compared to a structurally similar compound. These results confirm molecularly imprinted core-shell particles of the invention may be used for the separation of structurally similar compounds.

**Table 3:**

| Fractional uptake (U) for analogue pairs | | | | |
|---|---|---|---|---|
| Competitive binders | Polymer Imprint | U₁ | U₂ | Selectivity Ratio |
| caffeine/theophylline | P(caff) | U_{caff} = 0.94 | Uₜₕₑₒ = 0.51 | 1.84 |
| caffeine/theophylline | P(non-imprint) | U_{caff} = 0.56 | Uₜₕₑₒ = 0.51 | 1.10 |
| imidazole/piperazine | P(imid) | U_{imid} = 1.00 | Uₚᵢₚ = 0.20 | 5.00 |
| Imidazole/piperazine | P(non-imprint) | U_{imid} = 0.85 | Uₚₗₚ = 0.56 | 1.52 |
| propranolol/atenolol | P(prop) | Uₚᵣₒₚ = 0.94 | Uₐₜₑₙ = 0.27 | 3.48 |
| propranolol/atenolol | P(non-imprint) | Uₚᵣₒₚ = 1.00 | Uₐₜₑₙ = 1.00 | 1.00 |

### Example 9

The effect of the type of monomer used to construct the shell of core-shell particle emulsions was investigated when surface molecular imprinting was carried out using caffeine and theophylline template molecules in the two-step emulsion polymerisation procedure described in Example 1.

Core particles were prepared by forming a 1:1 DVB cross-linked polystyrene core with SDS as surfactant and aqueous 4-morpholineethane sulphonic acid monohydrate buffer at pH 6.0. Core-shell particle emulsions were prepared by surface template polymerisation of caffeine and theophylline in the presence of oleyl phenyl hydrogen phosphate (OPHP) and a monomer selected from ethylene glycol dimethacrylate (EGDMA), methacrylic acid (MA), Methylmethacrylate (MMA) and Styrene (ST). Blank (non-imprint) particles were prepared by carrying out the two-step emulsion polymerisation procedure in the absence of template molecule. Solid extraction cartridges containing the imprinted core-shell particles were prepared as described in Example 5 and the binding of caffeine and theophylline determined as described in Example 6. The findings of this study are reported in Table 4. A high selectivity ratio (1.84) is only observed when the shell components are EGDMA/OPHP and selectivity approaches zero when using other components. Consequently a shell polymer layer constructed from EGDMA and OPHP is advantageous for the construction of molecularly imprinted core-shell particles of the invention.

**Table 4:**

| Effect of shell composition on the selective uptake of caffeine over theophylline in core-shell particle resin | | | | | | |
|---|---|---|---|---|---|---|
| Shell (10nm) Monomer Composition | Fractional Uptake (U) by imprinted resin | | | | | |
| | P(caff) | | P(theo) | | P(blank) | |
| | U_{caff} | Uₜₕₑₒ | U_{caff} | Uₜₕₑₒ | U_{caff} | Uₜₕₑₒ |
| EGDMA/OPHP (SDS) | 0.94 | 0.51 | 0.57 | 0.48 | 0.56 | 0.51 |
| MA/OPHP (SDS) | 0.85 | 0.71 | 0.94 | 0.80 | 0.86 | 0.71 |
| MMA/OPHP (SDS) | 0.93 | 0.92 | 0.88 | 0.85 | 0.69 | 0.71 |
| ST/OPHP (SDS) | 0.90 | 0.81 | 0.91 | 0.79 | 0.90 | 0.80 |

## Claims

1. A particle capable of specific binding to an imprint molecule, the particle having an outer shell including cavities that correspond to the imprint molecule, and an inner core substantially free of such cavities, and which includes an amphipathic material.

2. A particle according to claim 1, which has polar surface groups.

3. A particle according to either preceding daim, wherein the shell is obtainable by polymerisation of ethylene glycol dimethacrylate and oleyl phenyl hydrogen phosphate.

4. A particle according to any preceding claim, wherein the cavities are formed by use of a soluble imprint molecule.

5. A particle according to any preceding claim, wherein the outer shell has a thickness of 1 to 100 nm.

6. Use of particles according to any of daims 1 to 5, as a medium for separation of a target molecule that is bound thereby.

7. Use of particles according to any of claims 1 to 5, as a medium for identification, purification, quantification or characterisation of a target molecule that is bound thereby.

8. A method of manufacturing particles according to any of claims 1 to 5, comprising the steps of:
a) polymerising a mixture of the imprint molecule and polymerisable material, around the core, to form a shell containing the imprint molecule; and
b) removing the imprint molecule, to leave a corresponding cavity;
wherein the amphipathic material is included in the mixture or the core is formed around a molecular scaffold which comprises the amphipathic material.

9. A method according to claim 8, wherein step (a) is conducted in an aqueous medium.

10. A method according to claim 8 or claim 9, wherein step (b) comprises washing.

## Patentansprüche

1. Teilchen, das sich spezifisch an ein Abdruckmolekül binden kann, wobei das Teilchen eine äußere Schale, die Hohlräume einschließt, welche dem Abdruckmolekül entsprechen, und einen inneren Kern aufweist, der im Wesentlichen frei von derartigen Hohlräumen ist, und ein amphipathisches Material einschließt.

2. Teilchen nach Anspruch 1, das polare Oberflächengruppen aufweist.

3. Teilchen nach einem vorangehenden Anspruch, in dem die Schale durch Polymerisation von Ethylenglycoldimethacrylat und Oleylphenylhydrogenphosphat erhältlich ist.

4. Teilchen nach irgendeinem vorangehenden Anspruch, in dem die Hohlräume durch Verwendung eines löslichen Abdruckmoleküls gebildet werden.

5. Teilchen nach irgendeinem vorangehenden Anspruch, in dem die äußere Schale eine Dicke von 1 bis 100 nm aufweist.

6. Verwendung von Teilchen nach irgendeinem der Ansprüche 1 bis 5 als Medium zur Abtrennung eines Zielmoleküle, das dadurch gebunden wird.

7. Verwendung von Teilchen nach irgendeinem der Ansprüche 1 bis 5 als Medium zur Identifikation, Reinigung, Quantifizierung oder Charakterisierung eines Zielmoleküls, das dadurch gebunden wird.

8. Verfahren zur Herstellung von Teilchen nach irgendeinem der Ansprüche 1 bis 5, welches die Schritte umfasst:
a) Polymerisieren einer Mischung des Abdruckmoleküls und von polymerisierbarem Material um den Kern herum, um eine Schale zu bilden, welche das Abdruckmolekül enthält; und
b) Entfernen des Abdruckmoleküls, um einen entsprechenden Hohlraum zurückzulassen;
wobei das amphipathische Material in die Mischung eingeschlossen wird oder der Kern um ein molekulares Gerüst herum gebildet wird, welches das amphipathische Material umfasst.

9. Verfahren nach Anspruch 8, in dem der Schritt (a) in einem wässrigen Medium durchgeführt wird.

10. Verfahren nach Anspruch 8 oder Anspruch 9, in dem der Schritt (b) ein Waschen umfasst.

## Revendications

1. Particule capable de liaison spécifique à une molécule d'impression, la particule ayant une enveloppe externe comprenant des cavités qui correspondent à la molécule d'impression, et un noyau interne sensiblement dépourvu de telles cavités, et qui comprend une matière amphipathique.

2. Particule selon la revendication 1, qui a des groupes de surface polaires.

3. Particule selon l'une ou l'autre des revendications précédentes, dans laquelle l'enveloppe peut être obtenue par polymérisation de diméthacrylate d'éthylèneglycol et d'hydrogénophosphate de phényle et d'oléyle.

4. Particule selon l'une quelconque des revendications précédentes, dans laquelle les cavités sont formées en utilisant une molécule d'impression soluble.

5. Particule selon l'une quelconque des revendications précédentes, dans laquelle l'enveloppe externe a une épaisseur de 1 à 100 nm.

6. Utilisation de particules selon l'une quelconque des revendications 1 à 5, comme milieu pour la séparation d'une molécule cible qui est liée par ces particules.

7. Utilisation de particules selon l'une quelconque des revendications 1 à 5, comme milieu pour l'identification, la purification, le dosage ou la caractérisation d'une molécule cible qui est liée par ces particules.

8. Procédé de fabrication de particules selon l'une quelconque des revendications 1 à 5, comprenant les étapes suivantes :
a) polymériser un mélange de la molécule d'impression et d'une matière polymérisable, autour du noyau, pour former une enveloppe contenant la molécule d'impression ; et
b) éliminer la molécule d'impression pour laisser une cavité correspondante ;
dans lequel la matière amphipathique est incluse dans le mélange ou bien le noyau est formé autour d'une charpente moléculaire qui comprend la matière amphipathique.

9. Procédé selon la revendication 8, dans lequel l'étape (a) est conduite dans un milieu aqueux.

10. Procédé selon la revendication 8 ou la revendication 9, dans lequel l'étape (b) comprend un lavage.
